Europäisches Patentamt

European Patent Office

Office européen des brevets

(11) Publication number: **0 153 741**
**A2**

## (12) EUROPEAN PATENT APPLICATION

(21) Application number: **85102172.5**

(22) Date of filing: **27.02.85**

(51) Int. Cl.⁴: **A 61 B 5/08**

(30) Priority: **29.02.84 IT 4153484**

(43) Date of publication of application:
**04.09.85 Bulletin 85/36**

(84) Designated Contracting States:
**AT BE CH DE FR GB IT LI LU NL SE**

(71) Applicant: **Torresin, Giuseppe**
**Via Praarie, 28,**
**I-35010 San Giorgio Delle Pertiche (Padova)(IT)**

(72) Inventor: **Torresin, Giuseppe**
**Via Praarie, 28,**
**I-35010 San Giorgio Delle Pertiche (Padova)(IT)**

(74) Representative: **KUHNEN & WACKER**
**Patentanwaltsbüro**
**Schneggstrasse 3-5 Postfach 1729**
**D-8050 Freising(DE)**

(54) **Apparatus for the analysis of the cardiorespiratory function at rest as well as during exercise.**

(57) An apparatus (1) for the analysis of the cardiorespiratory function at rest as well as during exercise comprises a mouthpiece (2), a control valve means (5), a spirometer (12) and an analysing and recording system (13). The mouthpiece (2) is connected via said control valve means (5) to said spirometer means (12) by conduit means (4). Furthermore, said analysing and recording system (13) is functionally connected to said spirometer means (12). To achieve the advantageous effects of the present invention there is provided a second control valve (10) which is disposed in said conduit means (7) between said first control valve means (5) and said spirometer means (12). Furthermore, there is provided a by-pass conduit (22), one end of which is connected to said conduit means (4) between said mouthpiece (2) and said control valve means (5) and the other end of which is connected to said second control valve means (10). The second control valve means (10) includes a diaphragm (14) which is adapted to open and close a line (16) in accordance with pressure conditions in said by-pass conduit (22) which directly can be changed by the patient's inhaling and exhaling, respectively. This arrangement ensures a simple and reliable design and offers a wide range of possibilities for medial applications.

0153741

G. Torresin

I-35011 S. Giorgio Delle Pertiche

## Apparatus For The Analysis of the Cardio-respiratory Function At Rest As Well As During Exercise

The invention refers to an apparatus for the analysis of the cardiorespiratory function at rest as well as during exercise, according to the opening part of claim 1.

Such an apparatus is, for example, known from British Patent 12 52 626. This apparatus comprises a mouthpiece, a control valve means, a spirometer means and an analysing and recording system. The mouthpiece used by the patient is disposed in conduit means and connected via said control valve means consisting of two flap valves to said spirometer means. The analysing and recording system is connected to said spirometer means by a counter weight, counter-balancing the bell chamber of the spirometer with the analysing and recording system consisting of a kymograph.

Furthermore, the bell chamber of the spirometer is divided into two equal volume sub-chambers, one of which being in the form of an annulus surrounding the other sub-chamber. Two airways are provided for each sub-chamber with two of these airways leading to atmosphere via electrically controlled valves and with the remaining airways leading to said mouthpiece via said flap valves. Moreover, there is provided a line connected to one of the airways immediately downstream of the expiratory flap valve and leading to an apparatus for sampling gases and another line between the expiratory and inspiratory flap valve leading to a pressure-sensitive device for operation of said electrically controlled valves. These latter valves enable on one hand a controlled collecting of the exhaled gases in one of the sub-chambers and on the other hand the replenishing of the other sub-chamber with fresh air during the operation of the apparatus.

One of the basic disadvantages of this known apparatus lies in its complicated arrangement concerning the design of the bell chamber, the necessity of a plurality of airways and electrically controlled valves which, moreover, have to be operated by said pressure-sensitive device. This complex arrangement leads to a raised price for the whole system and lowers its reliability because of a lot of technically complicated parts each of which has to be in order to ensure a perfect operation of the system.

A further problem resides in the different temperatures of the expired gas and the stored gas respectively leading to an unavoidable error of the gas volumes to be measured.

An another complicated system is described in US-Patent 37 99 149 to Rummel et al. Rummel teaches the use of valves in the inspiration and expiration lines which are controlled by a complex electrical circuit and not by the breathing action itself.

US-Patent 34 03 678 to Bolie describes a spirometer with ventilation flowmeters which is a different approach with reference to the above mentioned spirometer.

It is therefore the problem underlying the present invention to provide an apparatus for the analysis of the cardiorespiratory function at rest as well as during exercise, according to the opening part of claim 1 which provides a simple and reliable design offering, however, a wide range of operations concerning the medical test program.

The solution of this problem is achieved by the characterizing features of claim 1.

With the disposition of a second control valve means between the first control valve means and the spirometer means and the provision of a by-pass conduit connecting the part of the main conduit immediately downstream of the mouthpiece with the second control valve means, the whole apparatus can be controlled only by the change of the pressure conditions in said by-pass conduit with the change of the pressure conditions being effected by the patient's inhaling and exhaling, respectively. With the exception of the second control valve means and the by-pass conduit there is no necessity for further complicated control means to ensure the perfect function of the apparatus according to invention. The reliability and low-cost  construction of the apparatus according to the invention is further improved by the fact that the by-pass conduit and the additional control valve means form inexpensive parts of a simple and reliable design that replace all of the complicated and expensive control members of the apparatus according to the generic state of the art.

Inspite of its simple design the apparatus according to the invention provides all capabilities for the realization of the wide range of medical tests like ventilation, diffusion, perfusion studies and metabilic study applications as cardiopulmonary stress testing, intensive therapy, post-operating or post-infarcting clinic rehabilitation, working capability evaluation, athletic and risk evaluation in sports medicine, cardiovascular and pharmacological research, nutritional studies and environmental stress.

Further advantages result from the subclaims containing preferred embodiments of the invention.

By the provision of a usual non-rebreathing valve according to claim 2, a fast diaphragm valve according to claim 3 and a light bell spirometer without counterweight according to claim 7 the simplicity, reliability constant measuring characteristics and low-cost design of the apparatus according to the invention is furthermore improved.

Moreover, the spirometer is in line with the CECA standards (standarized lung function testing, report working party, ed Quanjer, European Commission for Coal and Steel, Luxembourg, July 83, Bull. de Physiopat, Resp. suppl. 5, vol. 19, 1983). The advantage of the provision of a spirometer according to the CECA standards is that these standards are more restrictive than the ATS standards (statements, Am. Rev. Resp. Dis., vol. 119, pg. 831, 1978). Especially, the spirometer of the apparatus according to the invention has a back-pressure that is lower than 0.1 KPa.s/l when the flow is in the range of 12 ltr/sec.

With the preferred embodiment according to the claims 8 thru 11 the apparatus according to the invention provides the possibility of a wide range of operations that can be visualized on a terminal. With these operations a computer system according to the invention can be processed in two different ways, one of which provides an immediate test control and the other one of which, at a later time, offers the possibility of an intensive study and an exact interpretation of all medical data explored and stored during operation. During the immediate test control the computer processes and monitors the three channel electrocardiograph at a 150 Hz frequency, the $CO_2$-production, the $O_2$-consumption, the ventilation and pulse trends as well as cardiac frequency values and the respiratory ratio. The visualization of these parameters allows the operator to check, at any time, the patient's health conditions and, possibly, to defer the test. At the end of the test the data which have been picked up every four seconds, are stored into a minidisc of e.g. 320 KByte which may carry the results of 300 tests. The interpretation and analysis of the tests can be recalled at any time.

In the subsequent data processing stage the computer provides the possibility of visualization and a printout of average data as load $\underline{/\bar{\phantom{W}}W\underline{\phantom{/}}/}$, $O_2$-consumption, $CO_2$-production, ventilation, pulse respiratory frequency, respiratory ratio, $O_2$-respiratory equivalent, $O_2$/weight, $O_2$/pulse, maximum voluntary ventilation and their trends, vs. time, alternative, vs. any other parameter. These data may be visualized by overimposition to permit a quick and easy comparison of them. Moreover, it is possible to reconstruct the graphics of $CO_2$-production, $O_2$-consumption, pulse and ventilation that have been monitored during the test for different mathematical or research elaboration.

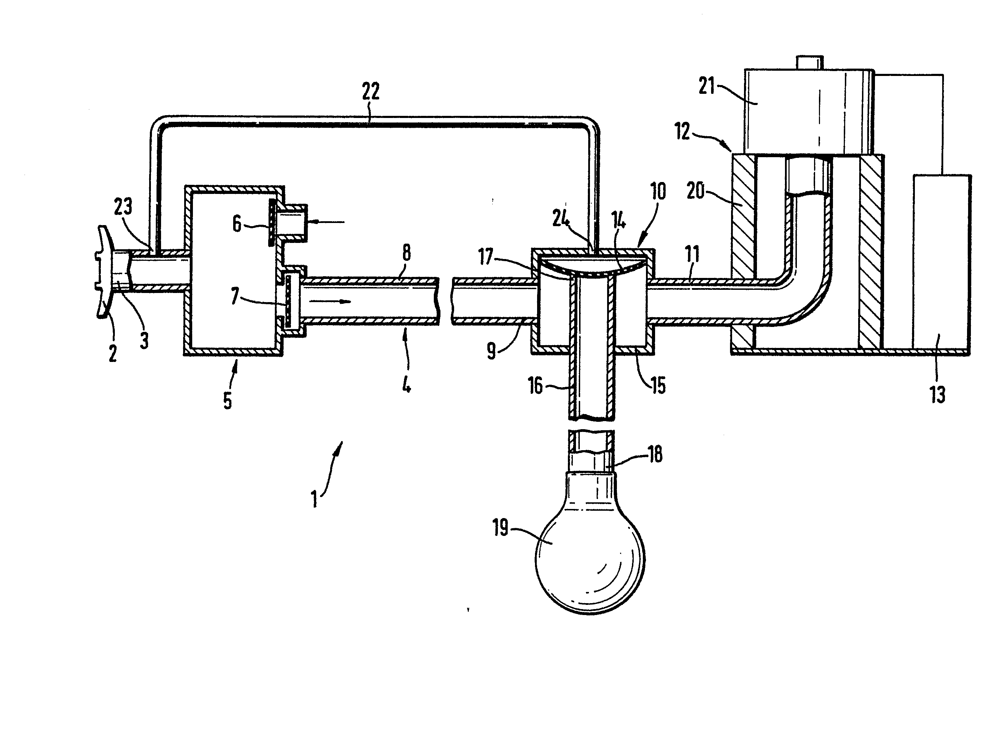

Various other advantages and features of the invention will become apparent from the following description taken in conjunction with the accompanying drawings, in which the only figure shows a schematic view of the apparatus according to the invention.

An apparatus 1 for the analysis of the cardiorespiratory function at rest as well as during excercise embodying the various features of the invention is schematically illustrated in the drawings in which numeral 2 designates a mouthpiece which is disposed at one end 3 of a conduit 4. A first control valve means 5 is disposed downstream of said mouthpiece 2 in said conduit 4 and consists, in the embodiment shown, of a non-rebreathing valve including e.g. two flap valves 6 and 7, respectively. Flap valve 6 leads to atmosphere and flap valve 7 is connected to an intermediate part 8 of said conduit 4 with the opposing end 9 of said intermediate part 8 being connected to a second control valve means 10.

Said control valve means 10 is connected via a third part 11 of said conduit 4 to a spirometer means 12 that is functionally connected to an analysing and recording system 13, which, as all other parts of the embodiment shown, is only illustrated in a simplified manner.

Said second control valve means 10 includes preferably a diaphragm valve comprising a diaphragm 14 disposed in a housing 15 and being adapted to open and close a line 16. Said line 16 is fixed in said housing 15 and one end 17 of said line 16 is disposed such that said diaphragm 14 may close said end 17 under certain pressure condition and may open said end 17 if these pressure conditions are appropriately changed. The other end 18 of said line 16 is connected to a sampling bag 19 for exhaled air. In the preferred embodiment shown the diaphragm valve is a quickly discharging three-way valve with an interal diameter of 50 mm and an external diameter of 100 mm. Furthermore, the diameter of line 16 is 50 mm in order to obtain an emptiness speed of more than 6.6 ltr/sec and an efficient monitoring of 200 ltr/min ventilation.

In the preferred embodiment shown in the drawing said spirometer means 10 includes a light bell spirometer without counterweight. Said third part 11 of conduit 4 leads into the housing 20 of said spirometer means 12 and has an internal diameter of 50 mm. The bell 21 has a weight of 150 gr and a diameter of 230 mm. By this arrangement the spirometer means 12 provides a very low back-pressure which is almost completely independent of the position of the bell.

Furthermore, there is provided a by-pass conduit 22, one end 23 of which is connected to said conduit 4 between said mouthpiece 2 and said first control valve means 5. The other end 24 of said conduit 22 is connected to said second control valve means 10 in such a manner that said diaphragm 14 may be controlled in its position with respect to said end 17 of said line 16 by the pressure conditions in said by-pass conduit 22.

Furthermore, said analysing and recording system 13 comprises in detail a signal transducer (signal monitoring potentiometer), an analyzer for $CO_2$ in the exhaled air (time constant of 100 msec), an analyzer for $O_2$ in the exhaled air (time constant of either about 180 msec or about 5-10 sec) a threechannel electrocardiograph, a data acquisition interface with an analogical/digital converter, a graphic capabilities computer and floppy discs for the medical data storage and data processing and a graphic capabilities printer, with all of these parts being represented in the drawing by one box being designated by numeral 13.

The apparatus 1 according to the invention operates as follows:

When the patient exhales, air passes through mouthpiece 2 and thru said conduit 4 via said first control valve means 5, said second control valve means 10 into said spirometer means 12, thereby closing flap valve 6 and opening flap valve 7 by positive pressure effected by the air exhaled. At the same time also positive pressure is built up in said by-pass conduit 22 with said increased positive pressure acting on said diaphragm 14 and moving it into a position in which said end 17 of said line 16 is closed. Under these conditions the exhaled air reaches said spirometer means 12, moves the bell 21 and the corresponding data may be monitored, analysed and stored by said analysing and recording system 13.

When, however, the patient inhales, the pressure in conduit 4 as well as in said by-pass conduit 22 is decreased. By this depression said flap valve 6 is opened while said flap valve 9 is closed so that the patient can draw fresh air into his lungs through said flap valve 6 and said mouth piece 2.

Furthermore, during inhaling, also the pressure in said by-pass conduit 22 drops and makes said diaphragm 14 move away from its sealing seat on said end 17 of said line 16 thereby opening said line 16 and enabling the air contained inside said spirometer means 12 to flow into said sampling bag 19. This process, too, may be monitored, analysed and stored by said analysing and recording system. Moreover, the above described processes may be repeated as often as necessary for the medical tests to be worked out.

During the operation the signal transducer sends the ventilation signal to the computer while the expired air collected in the sampling bag 19 is analysed and the relative signal is also sent to perform the metabolism, ventilation, diffusion evaluations together with a cardiac frequency and electrocardiogram.

The apparatus according to the invention most of all includes the advantage of a simple design and a high reliability and is adapted to cover a wide field of applications. Furthermore, a continuous analysis of gas and a correlation with respect to ventilation heart rate and other physiological parameters, e.g. pH oxygen and carbon dioxide concentration in blood can be effected.

Moreover, an analysis of the expired air can be cheaply executed by a slow analyzer analysing the contents of said sampling bag. A direct analysis may be effected directly from the patient's mouth using an analyzer having two levels, i.e. a fast analysing level for research purposes and a slow analysing level for clinical purposes.

For the determination of the volume and the morphologic features of the expired gas, the air volume of the patient's mouth is multiplied by the ventilation signal (corrected by the time delay because of sampling line). Furthermore, the ECG-signal is taken from the electrocardiograph. For a better understanding during the procedure of the investigation, different paramters may be displayed in a different form on the same computer monitor. Then the computer monitors quickly the ECG and slowly the ventilation, pulse signals, $O_2$-con- sumption and $CO_2$-production as well as quantitative variations of other gases according to the directions described as follows:

The signals to the computer may be processed in two different ways:

In real time for an immediate test control and, in postponed time, for an intensive study and a more careful interpretation.

In real time the computer processes and monitors the three-channel electrocardiograph at a 200 Hz frequency, the $CO_2$-production, the $O_2$-consumption, the ventilation and pulse trends, as well as cardiac frequency values and respiratory ratio.

The visualization of these parameters allows the operator to control at any time, the patent's health conditions and, possibly, to decide to interrupt the test.

For processing in real time a large amount of signals and a higher level language have been used.

At the end of the test the data that have been picked up every four seconds, are memorized in a minidisc of e.g. 320 KByte, which may store about 40 tests completely.

The signals stored in the computer may be visualized or printed cut as graphical or numerical data. For clinical purposes, only mean values/minute are used, whereas exact data every 4 seconds may be provided for the research.

So, the interpretation and analysis of the tests may also be executed at a later time simply by recalling the respective data stored.

Also in this subsequent data processing stage the computer furnishes the operator with a possibility of visualizing and printing the average data as the load $\underline{/}W\underline{/}$, the $O_2$-consumption, the $CO_2$-production, the ventilation, the pulse, the respiratory frequency, the respiratory ratio, the $O_2$-respiratory equivalent, the $O_2$/weight, the $O_2$/pulse, the maximum voluntary ventilation and their trends vs. time or, alternatively, vs. any other parameter.

G. Torresin

I-35011  S. Giorgio Delle Pertiche

0153741

## C L A I M S

1. An apparatus for the analysis of the cardiorespiratory function at rest as well as during exercise comprising

   a mouth piece (2),

   a control valve means (5),

   a spirometer means (12), and

   an analysing and recording system (13)

   with said mouthpiece being connected via said control valve means to said spirometer means by conduit means and with said analysing and recording system being connected to said spirometer means, being characterized in that a second control valve means (10) is disposed in said conduit means (4) between sai: first control valve means (5) and said spirometer means (12) and that there is provided a by-pass conduit (22), one end of which being connected to said conduit means (4) between said mouth piece (2) and said first control valve means (5) and the other end of which being connected to said second control valve (10), said first control valve means (5) is a non-rebreathing valve and said second control valve means (10) includes a diaphragm 614) being adapted to open and close a line (16) for exhaled air corresponding to the pressure conditions in said by-pass conduit (22).

2. An apparatus as claimed in claim 1, being characterized in that said second control valve means (10) is a quickly discharging three-way diaphragm valve.

3. An apparatus as claimed in claim 1,
   being characterized in that said spirometer means
   (12) is a light bell spirometer without counterweight.

4. An apparatus as claimed in claim 1, being character-
   ized in that said line (16) is connected to a
   sampling bag (19).

5. An apparatus as claimed in one of claims 1 thru 4,
   being characterized in that said analysing and
   recording system (13) includes analyzers for $CO_2$
   and/or $O_2$ in the exhaled air with said analyzers
   having a time constant of 100 msec.

6. An apparatus as claimed in one of claims 1 thru 5,
   being characterized in that said analysing and
   recording system further includes a three-channel
   electrocardiograph.

7. An apparatus as claimed in one of claims 1 thru 6,
   being characterized in that said analysing and
   recording system includes a data acquisition interface  with an analogical/digial converter.

8. An apparatus as claimed in one of claims 1 thru 7,
   being characterized in that said analysing and recording system further includes graphic capabilities
   computer means and floppy disc means for the medical
   data storage and data processing and/or graphic
   capabilities printer means.

0153741